(19) Europäisches Patentamt
European Patent Office
Office européen des brevets

(11) Veröffentlichungsnummer : **0 208 952**
**B1**

(12) # EUROPÄISCHE PATENTSCHRIFT

(45) Veröffentlichungstag der Patentschrift : **20.09.89**

(51) Int. Cl.$^4$ : **G 01 N 33/52**// C12Q1/34, C12Q1/28

(21) Anmeldenummer : **86108502.5**

(22) Anmeldetag : **21.06.86**

(54) **Testträger.**

(30) Priorität : **29.06.85 DE 3523439**

(43) Veröffentlichungstag der Anmeldung : **21.01.87 Patentblatt 87/04**

(45) Bekanntmachung des Hinweises auf die Patenterteilung : **20.09.89 Patentblatt 89/38**

(84) Benannte Vertragsstaaten :
**AT BE CH DE FR GB IT LI LU NL SE**

(56) Entgegenhaltungen :
**EP-A- 0 045 476**
**EP-A- 0 113 896**
**EP-A- 0 133 895**
**DE-A- 2 446 968**

(73) Patentinhaber : **BOEHRINGER MANNHEIM GMBH**
**Sandhofer Strasse 116**
**D-6800 Mannheim 31 (DE)**

(72) Erfinder : **Berger, Dieter, Dr. rer. nat.**
**Bensheimer Strasse 45**
**D-6806 Viernheim (DE)**
Erfinder : **Biegel, Elke**
**Fritz-Walter-Strasse 27**
**D-6800 Mannheim 51 (DE)**
Erfinder : **Frey, Günter**
**Pfalzgrafenstrasse 7**
**D-6701 Ellerstadt (DE)**
Erfinder : **Murawski, Hans-Rüdiger, Dr. rer. nat.**
**Carl-Lepper-Strasse 10**
**D-6840 Lampertheim (DE)**

**EP 0 208 952 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft einen Testträger, wie er insbesondere für die Bestimmung von Bestandteilen von Körperflüssigkeiten von Menschen und Tieren vielfache Verwendung findet.

Während früher im klinischen Labor die Konzentrationen beispielsweise der Bestandteile des Blutes, praktisch ausschließlich mit Hilfe flüssiger Reagenzien bestimmt wurden, haben in jüngerer Zeit sogenannte trägergebundene Tests zunehmend an Bedeutung gewonnen. Bei diesen sind die Reagenzien in entsprechenden Schichten eines festen Testträgers eingebettet, auf den ein Tropfen von beispielsweise Blut aufgebracht wird. Die darin enthaltenen Inhaltsstoffe führen zu einem charakteristischen Farbumschlag der Reagenzien auf dem Testträger. Dieser Farbumschlag wird entweder unmittelbar visuell ausgewertet oder mit Hilfe eines Gerätes, meistens reflexionsphotometrisch, gemessen.

Derartige Testträger sind häufig als Teststreifen ausgebildet, die im wesentlichen aus einer länglichen Trägerschicht aus Kunststoffmaterial und darauf angebrachten Testfeldern bestehen. Es sind jedoch auch Testträger bekannt, die als quadratische oder rechteckige Plättchen gestaltet sind.

Die Testträger bestehen vielfach aus mehreren Schichten, die in verschiedenerlei Weise miteinander verbunden sind. Die Verbindung wird häufig durch Kleben oder Schmelzkleben erzeugt. Bei anderen bekannten Testträgern werden mehrere Schichten ähnlich den Schichten eines photographischen Films unmittelbar aufeinander aufgebracht. Obwohl diese Verbindungsverfahren für verschiedenerlei Zwecke befriedigend sind, lassen sie in anderer Hinsicht zu wünschen übrig. Insbesondere, wenn mehrere Schichten, zum Beispiel aus produktionstechnischen oder konstruktiven Gründen nicht vollflächig verklebt oder verbunden werden können, tritt eine Flüssigkeit unter Umständen nicht schnell und vollständig genug aus einer Schicht in eine andere Schicht über.

Der Übergang einer Flüssigkeit in die einzelnen Schichten wird in einem solchen Fall durch einen aus dem US Patent 3,802,842 bzw. dem deutschen Patent 21 18 455 bekannten Vorschlag verbessert, bei dem die eigentlichen Reaktionsschichten nur lose auf einer Trägerschicht aufliegen und durch ein sie überspannendes, an seinen Rändern mit der Trägerschicht verbundenes Netz an dieser fixiert werden. Auch hier läßt jedoch der Flüssigkeitsübergang von Schicht zu Schicht zu wünschen übrig.

Aufgabe der vorliegenden Erfindung ist es daher, eine neuartige Verbindung der Schichten diagnostischer Testträger zur Verfügung zu stellen, die die genannten Probleme vermeidet und insbesondere den Flüssigkeitsübergang von Schicht zu Schicht nachhaltig födert.

Diese Aufgabe wird gelöst durch die in den Patentansprüchen gekennzeichnete Erfindung.

Bei der Analyse mit Hilfe von trägergebundenen Tests steht meist nur eine sehr geringe Flüssigkeitsmenge zur Verfügung. Dies gilt insbesondere, wenn ein durch einen Stich in den Finger oder das Ohrläppchen des Patienten gewonnener Blutstropfen zur Analyse ausreichen soll. Gerade in einem derartigen Fall kommt es besonders auf einen möglichst schnellen und vollständigen Übertritt der zu analysierenden Flüssigkeit zwischen den Schichten des Testträgers an.

Bei der Herstellung diagnostischer Testträger allgemein und insbesondere bei der Herstellung von Teststreifen werden hohe Anforderungen gestellt. Insbesondere handelt es sich um Massenprodukte, die in sehr hohen Stückzahlen produziert werden müssen. Überraschenderweise hat sich gezeigt, daß das Vernähen in diesen Herstellvorgang ohne besondere Probleme eingefügt werden kann. Die so hergestellten Verbindungen zwischen den einzelnen Schichten sind zuverlässig und langfristig haltbar. Flüssigkeiten können durch den Spalt zwischen den vernähten Schichten eindringen und die Schichten somit problemlos benetzen. Vor allem aber fördert die Vernähung, wie sich im Rahmen der vorliegenden Erfindung gezeigt hat, in erstaunlichem Maße den Flüssigkeitsübertritt zwischen den durch Nähen verbundenen Schichten. Dabei haben sich eine Vernähung mit monofilen Fäden gegenüber multifilen Fäden und wenig saugfähige Kunststoffmaterialien gegenüber meist saugfähigeren Naturstoffen (wie Baumwolle) als überlegene Fadenmaterialien erwiesen. Die den Flüssigkeitsübertritt födernde Wirkung der Fäden ist also offenbar nicht auf deren Saugvermögen (Dochtwirkung) zurückzuführen.

Die zu verbindenden Schichten müssen nicht gleich groß sein. Häufig werden vielmehr kleinflächige Schichten mit größeren verbunden, beispielsweise kleine Testfelder auf eine größere Unterlage aufgenäht. Dabei hat es sich als vorteilhaft erwiesen, wenn die Nähte einen Abstand von zwischen 0,5 und 3 mm, bevorzugt zwischen 1 und 2 mm vom jeweils nächstliegenden Rand einer Schicht haben. Die Dichte der Stiche sollte so beschaffen sein, daß sie voneinander einen Abstand von 0,2 bis 3 mm, bevorzugt 0,5 bis 2 mm haben, wenn man von einer einfachen geraden Naht ausgeht. Eine Zickzacknaht ist auch möglich, jedoch wegen des erhöhten Platzbedarfs weniger bevorzugt. Bei einem zu großen Abstand der Stiche voneinander wird kein ausreichender Flüssigkeitsübertritt zwischen den vernähten Schichten erreicht. Ist der Abstand der Stiche zu klein, so ergibt sich ein zu großer Strömungswiderstand in Richtung quer zur Naht. Außerdem nehmen die von den Nähnadeln erzeugten Löcher einen zu großen Teil der zu analysierenden Flüssigkeit auf.

Es können mehrere Nähte verwendet werden. Als besonders geeignet hat sich eine Anordnung erwiesen, bei der mindestens zwei Nähte etwa parallel zueinander in einem Abstand von höchstens 15 mm, bevorzugt höchstens 10 mm angeordnet sind. Die beiden Nähte sorgen zum Einen dafür, daß die Schichten weitgehend plan aufeinander liegen. Zum Zweiten tritt die Flüssigkeit an den jeweiligen Nähten aus einer Schicht in die andere schnell über und breitet sich dann von den beiden Nähten ausgehend gleichmäßig in der zu benetzenden Schicht aus.

Die erwähnten Maßnahmen bezüglich der Anordnung der Nähte führen insgesamt zu einer optimalen Befestigung bezüglich Zuverlässigkeit und schnellem Flüssigkeitsübertritt zwischen den Schichten.

Besondere Probleme bestehen bei Testträgern, insbesondere Teststreifen, die eine Trägerschicht aus einer steifen Kunststoffolie aufweisen. Die Kunststoffolie muß dabei so steif sein, daß der Teststreifen problemlos gehandhabt werden kann. Um auch bei derartigen Testträgern den Einsatz der neuartigen Nähtechnologie zu ermöglichen, wird gemäß bevorzugter Ausführungsform der Erfindung ein sandwichartiger Aufbau mit mindestens drei Schichten vorgeschlagen, bei dem die Reaktionsschicht nicht unmittelbar mit der Trägerschicht, sondern mit einer zwischengeschalteten Verbindungsschicht vernäht wird. Danach wird die Verbindungsschicht in konventioneller Weise, beispielsweise durch Schmelzkleben, mit der Trägerschicht verbunden. Die Verbindungsschicht besteht dabei aus einem Material, das einerseits weich genug ist, daß es problemlos von den Nähnadeln durchstochen werden kann und das andererseits fest genug ist, daß es von den Nähfäden nicht zerrissen wird. Außerdem muß es mit der Tragerfolie gut verklebbar sein. Bewährt hat sich beispielsweise ein Kunststoffmaterial, das unter dem Markennamen Depron von der Firma Kalle, Wiesbaden, Bundesrepublik Deutschland vertrieben wird.

Weitere Ausgestaltungen und Vorzüge der vorliegenden Erfindung werden im Rahmen der folgenden Beschreibung eines Ausführungsbeispiels anhand der Zeichnungen erläutert. Es zeigen :

Figur 1 eine vereinfachte perspektivsche Darstellung eines erfindungsgemäßen Testträgers.

Figur 2 eine Darstellung des Schichtaufbaus einer bevorzugten Ausführungsform der Erfindung in Seitenansicht.

Der in Figur 1 dargestellte Testträger 10 hat die prinzipielle Form eines konventionellen Teststreifens. Es handelt sich jedoch um ein mit den früheren Teststreifen kaum mehr vergleichbares hochwertiges Analysesystem. Auf einer Trägerschicht 12 aus einer Kunststoffolie ist ein Testbereich angeordnet, der in seiner Gesamtheit mit 14 bezeichnet ist. Es handelt sich in diesem Fall um einen Testträger, der Analysen unmittelbar aus Vollblut statt aus Plasma ermöglicht. Zur Abtrennung der Erythrozyten werden Glasfasern verwendet. Nähere Einzelheiten sind der europäischen Patentanmeldung mit der Veröffentlichungsnummer 45 476 bzw. dem US-Patent 4, 477, 575 zu entnehmen.

Der Testbereich 14 des Testträgers 10 läßt sich in eine Auftragzone 16 und in eine Analysezone 18 unterteilen. Die Auftragzone 16 wird von einem Schutznetz 19 überdeckt. In der Analysezone erkennt man eine Klappe 20. Die Schichten unter der Klappe sind mit Nähten 22 miteinander vernäht. Der Schichtaufbau des Testtragers ist in seiner Gesamtheit mit 24 bezeichnet. Einzelheiten sind der folgenden Beschreibung der Figur 2 zu entnehmen.

Figur 2 zeigt den Aufbau des Testbereichs 14 einer bevorzugten Ausführungsform eines erfindungsgemaßen Testträgers 10 in seitlicher Ansicht. Dabei wird ein Testaufbau, der zur Bestimmung von Creatinin geeignet ist, beschrieben. Ein derartiger Test stellt besondere Anforderungen an den Aufbau eines Testträgers, die erfindungsgemäß erfüllt werden. Die Erfindung ist jedoch auch für andere Bestimmungen geeignet.

Die Trägerschicht 12 besteht wie erwähnt aus einer Kunststoffolie, wie sie bei der Herstellung von Teststreifen gebräuchlich ist. Diese Folie muß für die Handhabung und Herstellung der Testträger ausreichend steif sein. Im Rahmen der Erfindung hat sich gezeigt, daß eine derartige Kunststoffolie nur mit sehr großen Schwierigkeiten unmittelbar vernäht werden kann.

Mittels eines Schmelzklebers 26 ist auf die Trägerschicht ein mehrschichtiges Sandwich aufgeklebt, das aus einer Verbindungsschicht 28, einer Flüssigkeitstransportschicht 30 einer Reaktionsschicht 32 und einer Niederhalterschicht 34 besteht. Diese vier Schichten sind mit Nähnähten 22 untereinander verbunden. Nach dem Vernähen werden sie insgesamt auf die Trägerschicht 12 aufgeklebt.

Während die Reaktionsschicht 32 und die Niederhalterschicht 34 sich nur im Bereich der Analysezone 18 erstrecken, erstreckt sich die Verbindungsschicht 28 und die Flüssigkeitstransportschicht 30 bis in die Auftragzone 16. In dieser Zone sind darüber eine Vorreaktionsschicht 36 eine Plasmagewinnungsschicht 38 und das Schutznetz 19 angeordnet. Sie sind mit einem Schmelzkleberstreifen (40) einseitig verklebt.

Im Folgenden wird zunächst auf Einzelheiten der erfindungsgemäßen Nähtechnologie eingegangen. Anschließend werden Besonderheiten des Creatinintests beschrieben, der im bevorzugten Ausführungsbeispiel dargestellt ist.

Wie oben erwähnt, hat das Vernähen von Testträgerschichten insbesondere den Vorteil, daß ein zuverlässiger Flüssigkeitsübertritt zwischen den Schichten des Testträgers in Richtung senkrecht auf ihre Fläche auch bei sehr kleinen Flüssigkeitsmengen gewährleistet wird. Im dargestellten Ausführungsbeispiel ist die Flüssigkeit Blutplasma, das in einer kleinen Menge von beispielsweise etwa 20 ul in der Auftragzone erzeugt wird. Zu diesem Zweck wird ein Blutstropfen auf das Schutznetz 19 aufgegeben, durchdringt die Plasmagewinnungsschicht 38, wobei die Erythrozyten abgetrennt werden und gelangt über die Vorreaktionsschicht 36 in die Flüssigkeitstransportschicht 30. In dieser wird sie durch Kapillarwirkung in der Zeichnung nach links in die Analysezone 18 transportiert. Einzelheiten sind der zitierten europäischen Patentanmeldung 45 476 zu entnehmen.

Die Flüssigkeitstransportschicht 30 besteht bevorzugt aus einem sehr feinen und damit mechanisch empfindlichen Material mit vielen dünnen Fasern und dazwischen angeordneten Hohlräumen, die eine gute Kapillarwirkung gewährleisten. Aus der Flüssigkeitstransportschicht soll die Flüssigkeit möglichst schnell und weitgehend in die Reaktionsschicht 32 übertreten. Dieser Flüssigkeitsübertritt wird

erfindungsgemäß dadurch gefördert, daß die beiden Schichten mit den Nähten 22 miteinander vernäht sind.

Die Nähtechnologie ist generell auch beim Vernähen von nur zwei Schichten eines Testträgers von Vorteil. Besonders bevorzugt ist jedoch insbesondere dann, wenn die Tragerschicht 12 eine Kunststoffolie ist, die so steif ist, daß sie sich nur sehr schwer durchnähen läßt, die Verwendung der zusätzlichen Verbindungsschicht 28. In diesem Fall wird die Verbindungsschicht mit der Reaktionsschicht vernäht und das so gebildete Sandwich auf die Trägerschicht aufgeklebt. Dabei ist bevorzugt, aber nicht notwendigerweise zwischen der Verbindungsschicht 28 und der Reaktionsschicht 32 die Flüssigkeitstransportschicht 30 als separate Schicht angeordnet. Stattdessen könnte auch die Verbindungsschicht selbst als Flüssigkeitstransportschicht ausgebildet sein. Geeignet wäre eine zum Vernähen ausreichend fest ausgebildete Kunststoffolie, die zumindest auf der in der Figur oberen Oberfläche eine kapillaraktive Oberflächenstruktur hat, so daß sie zugleich als Flüssigkeitstransportschicht dient. Geeignet wäre auch eine Ausbreitungsschicht, wie sie in der deutschen Patentschrift 23 32 760 bzw. dem US-Patent 3,992,158 beschrieben ist. In diesem Fall würde also der Gesamtschichtaufbau aus mindestens drei Schichten, nämlich der Trägerschicht, der Verbindungsschicht und der Reaktionsschicht bestehen, wobei Reaktionsschicht und Verbindungsschicht untereinander vernäht und gemeinsam auf die Trägerschicht aufgeklebt werden.

Die Reaktionsschicht kann beispielsweise ein die Reagenzien enthaltender Film oder ein mit Reagenzien imprägniertes Papier oder Vlies sein. Insbesondere in solchen Fällen, in denen die Reaktionsschicht aus einem im feuchten Zustand aufquellenden Material hergestellt ist, kann es zweckmäßig sein, eine zusätzliche Niederhalterschicht 34 aufzunähen. Diese kann beispielsweise als poröses Netz ausgebildet sein, das aus einem nicht saugenden in Flüssigkeit dimensionsstabilen Material, insbesondere aus Kunststoff besteht. Eine derartige Niederhalterschicht 34 ist in Figur 2 zusätzlich zu einem insgesamt schon vierschichtigen Aufbau vorgesehen. Sie kann aber auch bei jeder anderen Schichtfolge eine wertvolle Ergänzung der erfindungsgemäßen Nähtechnologie sein. Wenn die Niederhalterschicht aus einem imprägnierbaren Material besteht, kann sie mit Reagenzien imprägniert sein.

Für den besonders bevorzugten Fall, daß der erfindungsgemäße Testträgeraufbau für die Bestimmung von Creatinin verwendet wird, gilt folgendes :

Die Creatininbestimmung erfolgt nach folgendem bekannten Reaktionsschema :

$$\text{a, Creatinin} + H_2O \xrightarrow{\text{Creatininase}} \text{Creatin}$$

$$\text{b, Creatin} + H_2O \xrightarrow{\text{Creatinase}} \text{Sarcosin} + \text{Harnstoff}$$

$$\text{c, Sarcosin} + H_2O \xrightarrow[O_2]{\text{Sarcosin-OD}} \text{Glycin} + HCHO + HO_2$$

$$\text{d, } H_2O_2 + \text{Indikator}_{Red} \xrightarrow{\text{Peroxidase}} \text{Indikator}_{Oxid} + H_2O$$

Als Reaktionsschritt d) kommt bevorzugt die in der deutschen Patentanmeldung 34 33 946 beschriebene Indikatorreaktion in Betracht, wobei in diesem Fall alle Reagenzien mit Ausnahme der als Kuppler für den Indikator verwendeten Anilinphosphonsäure in Form eines Reagenzfilmes in der Klappe 20 enthalten sind. Unter der Plasmagewinnungsschicht 38, die aus einem Glasfaservlies besteht, welches den Bedingungen der zitierten europäischen Patentanmeldung 45 476 genügt, befindet sich als Vorreaktionsschicht 36 ein Creatin-Abtrennpapier, in dem alle Reagenzien zur Abtrennung von endogenem, also in der Probe vorhandenem Creatin enthalten sind. Dies ist notwendig, weil die Nachweisreaktion für das Creatinin ihrerseits Creatin als Zwischenprodukt hat und endogenes Creatin daher den Nachweis verfälschen würde.

Die Flüssigkeitstransportschicht 30 ist als Glasfasergewebe oder Glasfaservlies von geringer Festigkeit und guten Flüssigkeitstransporteigenschaften ausgebildet. Die Glasfasern erfüllen neben dem Transport des Plasmas den Zweck, daß sie eventuell in der Plasmagewinnungsschicht 38 nicht vollständig entfernte Erythrozyten auf dem Transportwege des Plasmas von der Auftragszone 16 in die Analysezone 18 festhalten.

Die Reaktionsschicht 32 wird bei diesem Test ebenfalls als Vorreaktionsschicht verwendet und enthält die gleichen Reagenzien wie die Schicht 36. Die Niederhalterschicht 34 dient zugleich als Komponente für die in der Klappe 20 wie oben erwähnt, nicht enthaltene Komponente Anilinphosphonsäure.

Eine Creatininbestimmung mit einem derartigen Testaufbau läuft wie folgt ab :

Auf das Schutznetz 19 werden 30 µl Blut aufdosiert. Das Blut dringt in die Plasmagewinnungsschicht 38 ein, wo die Erythrozyten vom Plasma separiert werden. Danach dringt das Plasma in das Creatin-Abtrennpapier 36. In diesem läuft folgende Reaktion ab :

a) Creatin + $H_2O$ $\xrightarrow{\text{Creatinase}}$ Sarcosin + Harnstoff

b) Sarcosin + $H_2O$ $\xrightarrow{\text{Sarc.-OD}}$ Glycin + HCHO + $H_2O_2$

c) $2H_2O_2$ $\xrightarrow{\text{Katalase}}$ $2H_2O + O_2$

Dabei ist wichtig, daß die Katalase nicht in den weiteren Test verschleppt wird, da sie die Nachweisreaktion stören würde. Sie wird deswegen an dem Creatin-Abtrennpapierträger fixiert.

Das mit den Creatin-Abtrennreagenzien vorinkubierte Plasma fließt infolge von Kapillarkräften durch die Flüssigkeitstransportschicht in den Bereich der Analysezone 18. Über die beiden Nähte wird das als Reaktionsschicht 32 verwendete zweite Creatinabtrennpapier mit Plasma benetzt. Die beiden entstehenden Plasmafronten laufen von den beiden Nähten aufeinander zu und füllen das Creatin-Abtrennpapier 32 vollständig. Wegen des großen Einflusses einer Störung durch Creatin, ist es bei einem derartigen Test besonders wichtig, daß die zur Creatinabtrennung verwendete Reaktionsschicht 32 schnell und vollständig benetzt wird. Hier hat sich die Vernähung als besonders hilfreiche Maßnahme erwiesen. Weiter dringt die Flüssigkeit auch zu der Niederhalterschicht 34 vor und löst die darin enthaltene Anilinphosphonsäure. Außerdem drückt die Niederhalterschicht 34 auf das darunterliegende Creatin-Abtrennpapier 32 und trägt zu dessen vollständiger Benetzung bei.

In diesem Stadium erfordert der Test, daß eine verhältnismäßig lange Vorinkubationszeit von in diesem Fall etwa 100 Sekunden eingehalten wird, die zur Abtrennung des endogenen Creatin notwendig ist. Erst danach darf die Reaktionsfilmklappe 20 aufgedrückt werden.

Um einen vorzeitigen Kontakt zwischen der Reaktionsfilmklappe 20 und der Niederhalterschicht 34 mit der darin enthaltenen Reaktionslösung zu verhindern, ist eine Schutzfolie 50 vorgesehen, die einseitig entlang einer quer über den Teststreifen laufenden Linie mittels einer der Nähte 22 mit dem Schichtaufbau 24 vernäht ist.

Nach Ablauf der Vorinkubationszeit zur Entfernung des endogenen Creatin wird die Reaktionsfilmklappe, bevorzugt durch eine geeignete Mechanik in einem Auswertegerät, blasenfrei und vollständig auf die Niederhalterschicht aufgedrückt. Der Farbumschlag findet statt und wird reflexionsphotometrisch ausgewertet.

**Patentansprüche**

1. Testträger (10) für die Bestimmung der Konzentration von Bestandteilen von Flüssigkeiten, insbesondere von Körperflüssigkeiten von Menschen und Tieren, mit mindestens zwei miteinander verbundenen Schichten (30, 32), dadurch gekennzeichnet, daß die Schichten durch Nähte (22) miteinander verbunden sind.

2. Testträger nach Anspruch 1, dadurch gekennzeichnet, daß die Nähte (22) aus monofilen Fäden bestehen.

3. Testträger nach Anspruch 1 oder 2, dadurch gekennzeichnet, daß Stiche der Nähte (22) einen Abstand von 0,2 bis 3 mm bevorzugt 0,5 bis 2 mm haben.

4. Testträger nach einem der vorhergehenden Ansprüche, dadurch gekennzeichnet, daß die Nähte (22) vom jeweils nächstliegenden Rand der verbundenen Schichten einen Abstand von höchstens 0,5 bis 3 mm, bevorzugt 1 bis 2 mm haben.

5. Testträger nach einem der vorhergehenden Ansprüche, dadurch gekennzeichnet, daß mindestens zwei Nähte, bevorzugt mit einem Abstand von höchstens 10 mm vorgesehen sind.

6. Testträger nach Anspruch 5, gekennzeichnet durch eine zusätzliche, auf der Oberfläche einer der vernähten Schichten (32) angeordnete Niederhalterschicht (34).

7. Testträger nach einem der vorhergehenden Ansprüche mit einer Trägerschicht (12) aus einer steifen Kunststoffolie und mindestens einer Reaktionsschicht (32), dadurch gekennzeichnet, daß zwischen der Trägerschicht (12) und der Reaktionsschicht (32) eine Verbindungsschicht (28) angeordnet ist, wobei die Verbindungsschicht (28) mit der Reaktionsschicht durch Nähen verbunden ist und die Trägerschicht mit der Verbindungsschicht in anderer Weise, insbesondere durch Kleben verbunden ist.

8. Testträger nach Anspruch 7, dadurch gekennzeichnet, daß die Verbindungsschicht (28) als Flüssigkeitstransportschicht ausgebildet ist.

9. Testträger nach Anspruch 7, dadurch gekennzeichnet, daß zwischen der Verbindungsschicht (28) und der Reaktionsschicht (32) eine Flüssigkeitstransportschicht (30) angeordnet ist.

10. Testträger nach einem der Ansprüche 7 bis 9, dadurch gekennzeichnet, daß er als Teststreifen ausgebildet ist.

**Claims**

1. Test carrier (10) for the determination of the concentration of components of liquids, especially of

body fluids of humans and animals, with at least two layers (30, 32) bound with one another, characterised in that the layers are bound with one another by stitches (22).

2. Test carrier according to claim 1, characterised in that the stitches (22) consist of monofilar threads.

3. Test carrier according to claim 1 or 2, characterised in that the holes of the stitches (22) have a distance of 0.2 to 3 mm., preferably of 0.5 to 2 mm.

4. Test carrier according to one of the preceding claims, characterised in that the stitches (22) have, from the particular closest lying edge of the bound layers, a distance of at most 0.5 to 3 mm., preferably of 1 to 2 mm.

5. Test carrier according to one of the preceding claims, characterised in that there are provided at least two stitches, preferably with a distance of at most 10 mm.

6. Test carrier according to claim 5, characterised by an additional holding-down layer (34) arranged on the surface of one of the stitched layers (32).

7. Test carrier according to one of the preceding claims with a carrier layer (12) of a stiff synthetic resin film and at least one reaction layer (32), characterised in that between the carrier layer (12) and the reaction layer (32) there is arranged a connecting layer (28), whereby the connecting layer (28) is connected with the reagent layer by stitches and the carrier layer is connected with the connecting layer in another way, especially by adhesion.

8. Test carrier according to claim 7, characterised in that the connecting layer (28) is formed as liquid transport layer.

9. Test carrier according to claim 7, characterised in that between the connecting layer (28) and the reaction layer (32) there is arranged a liquid transport layer (30).

10. Test carrier according to one of claims 7 to 9, characterised in that it is formed as test strip.

## Revendications

1. Support de test (10) pour la détermination des constituants de liquides, en particulier de liquides de l'organisme de l'homme et de l'animal, avec au moins deux couches (30, 32) reliées entre elles, caractérisé en ce que les couches sont liées ensemble par des coutures (22).

2. Support de test selon la revendication 1, caractérisé en ce que les coutures (22) sont constituées par des fils à brin unique.

3. Support de test selon la revendication 1 ou 2, caractérisé en ce que les piqûres des coutures (22) sont à une distance de 0,2 à 3 mm, de préférence de 0,5 à 2 mm.

4. Support de test selon l'une des revendications précédentes, caractérisé en ce que les coutures (22) sont à une distance au plus de 0,5 à 3 mm, de préférence de 1 à 2 mm chaque fois du bord le plus proche des couches liées.

5. Support de test selon l'une des revendications précédentes, caractérisé en ce qu'au moins deux coutures, de préférence à une distance de 10 mm au plus, sont prévues.

6. Support de test selon la revendication 5, caractérisé par une couche de maintien (34) supplémentaire, disposée à la surface d'une des couches cousues (32).

7. Support de test selon l'une des revendications précédentes avec une couche support (12) en feuille plastique rigide et au moins une couche réactionnelle (32), caractérisé en ce qu'entre la couche support (12) et la couche réactionnelle (32) est disposée une couche de liaison (28), la couche de liaison (28) étant reliée à la couche réactionnelle par une couture et la couche support étant reliée à la couche de liaison d'une autre façon, en particulier par collage.

8. Support de test selon la revendication 7, caractérisé en ce que la couche de liaison (28) est réalisée en tant que couche de transport du liquide.

9. Support de test selon la revendication 7, caractérisé en ce qu'entre la couche de liaison (28) et la couche réactionnelle (32) est disposée une couche de transport du liquide (30).

10. Support de test selon une des revendications 7 à 9, caractérisé en ce qu'il est réalisé sous forme de bandelette de test.

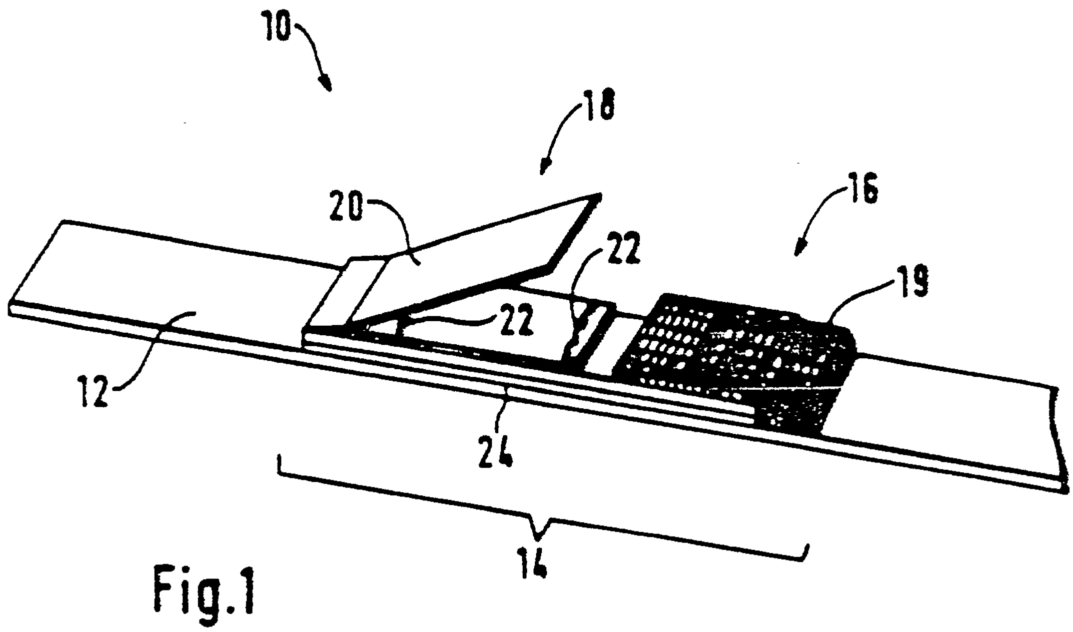
10
18
20
22
16
19
22
12
24
14

Fig.1

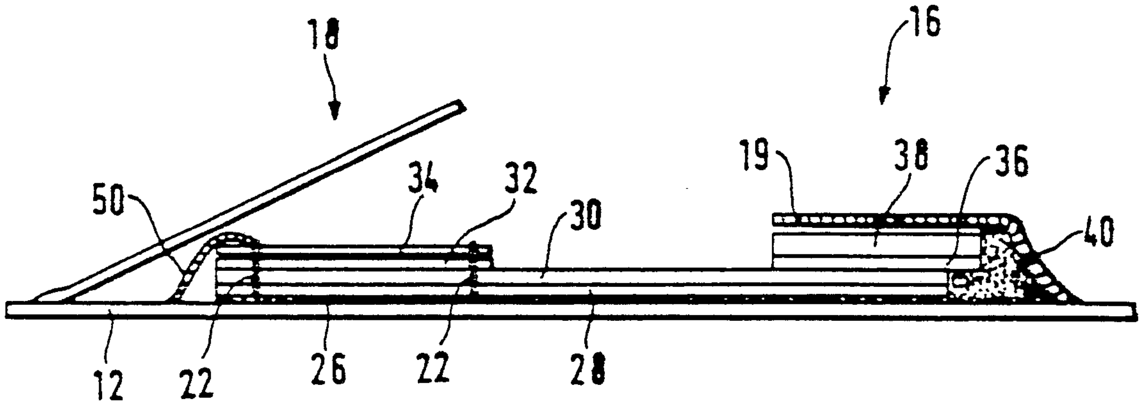
18
16
50
34
32
30
19
38
36
40
12
22
26
22
28

Fig.2